# EUROPEAN PATENT SPECIFICATION

(11) **EP 0 606 359 B1**
(45) Date of publication and mention of the grant of the patent: **08.04.1998**
(21) Application number: 92921166.2
(22) Date of filing: 30.09.1992
(51) Int. Cl.: A01H 5/10, A01H 1/02

(54) **CANOLA OIL WITH REDUCED LINOLENIC ACID AND LOW SULFUR**
CANOLAÖL MIT NIEDRIGEM LINOLENSÄURE- UND SCHWEFELGEHALT
HUILE DE CANOLA A TENEUR REDUITE EN ACIDE LINOLENIQUE ET SOUFRE

(30) Priority: 30.09.1991 US 767748
(43) Date of publication of application: 20.07.1994
(60) Divisional application: 97200546.6
(73) Proprietor: Cargill Incorporated, Wayzata, Minnesota 55391 (US)
(72) Inventor: DeBONTE, Lorin, R., Fort Collins, CO 80525 (US); LOH, Willie, H.-T., Minneapolis MN 55417 (US); FAN, Zhegong X, Fort Collins CO 80525 (US)
(74) Representative: Cockbain, Julian, Dr.
(86) International application number: US9208140
(87) International publication number: WO9306714

(56) References cited:
- EP-A- 0 323 753
- WO-A-90/10380
- WO-A-92/03919
- CANADIAN JOURNAL PLANT SCIENCE vol. 68, no. 4, April 1988, pages 509 - 511 R. SCARTH ET AL 'Stellar low linolenic-high linoleic acid summer rape'
- CANADIAN JOURNAL PLANT SCIENCE vol. 55, no. 1, January 1975, pages 343 - 344 B. R.STEFANSSON & Z.P.KONDRA 'Tower summer rape'
- JOURNAL AMERICAN OIL CHEMIST'S SOCIETY vol. 67, no. 3, March 1990, pages 161 - 164 A.PREVOT ET AL 'A new variety of low-linolenic rapeseed oil; characteristics and room odor tests'
- ZEITSCHRIFT PFLANZENZÜCHTUNG vol. 95, 1985, pages 201 - 209 N.N.ROY & A.W.TARR 'IXLIN - an interspecific source for high linoleic and low linolenic acid content in rapeseed (Brassica napus L.)'

## Description

This invention relates to improved canola seeds, plants and oil having advantageous properties, that is, a low glucosinolates content and a very low a-linolenic acid (C_{18:3}) content, which produce an oil with low sulfur content, improved sensory characteristics and oxidative stability.

### 1. Background

A need exists for an improved vegetable oil with a significantly extended shelf life and greater heat stability relative to generic canola oil and a positive nutritional contribution to animal, including human, diets.

Canola oil has the lowest level of saturated fatty acids of all vegetable oils. "Canola" refers to rapeseed (Brassica) which has an erucic acid (C221) content of at most 2 percent by weight based on the total fatty acid content of a seed, preferably at most 0.5 percent by weight and most preferably essentially 0 percent by weight and which produces, after crushing, an air-dried meal containing less than 30 micromoles (µmol) per gram of defatted (oil-free) meal. These types of rapeseed are distinguished by their edibility in comparison to more traditional varieties of the species.

As consumers become more aware of the health impact of lipid nutrition, consumption of canola oil in the U.S. has increased. However, generic canola oil cannot be used in deep frying operations, an important segment of the food processing industry

Canola oil extracted from natural and previously commercially useful varieties of rapeseed contains a relatively high (8%-10%) a-linolenic acid content (C_{18:3}) (ALA). This trienoic fatty acid is unstable and easily oxidized during cooking, which in turn creates off-flavors of the oil (Gailliard, 1980, Vol. 4, pp. 85-116 In: Stumpf, P. K., ed., The Biochemistry of Plants, Academic Press, New York). It also develops off odors and rancid flavors during storage (Hawrysh, 1990, Stability of canola oil, Chapter 7, pp. 99-122 In: F. Shahidi, ed. Canola and Rapeseed: Production, Chemistry Nutrition, and Processing Technology, Van Nostrand Reinhold, New York). One such unsatisfactory species heretofore has been Brassica napus, i.e., spring canola, a type of rapeseed.

It is known that reducing the a-linolenic content level by hydrogenation increases the oxidative stability of the oil. Hydrogenation is routinely used to reduce the polyunsaturates content of vegetable oils, thereby increasing its oxidative stability. The food industry has used hydrogenation to raise the melting point of vegetable oils, producing oil-based products with textures similar to butter, lard and tallow. Trans isomers of unsaturated fatty acids are commonly produced during hydrogenation. However, the nutritional properties of trans fatty acids mimic saturated fatty acids, thereby reducing the overall desirability of hydrogenated oils (Mensink et al., New England J. Medicine N323:439-445, 1990; Scarth, et al., Can. J. PI. Sci., 68:509-511, 1988). Canola oil produced from seeds having a reduced a-linolenic acid content would be expected to have improved functionality for cooking purposes with improved nutritional value, and therefore have improved value as an industrial frying oil.

However, in general, very little variation exists for a-linolenic acid content in previously known canola quality B. napus germplasm (Mahler et al., 1988, Fatty acid composition of Idao Misc. Ser. No. 125). Lines with levels of a-linolenic acid lower than that of generic canola oil are known, but have sensory, genetic stability, agronomic or other nutritional deficiencies. For example, Rakow et al. (J. Am. Oil Chem. Soc., 50:400-403, 1973), and Rakow (Z. Pflanzenzuchtg, 69:62-82, 1973), disclose two a-linolenic acid mutants, M57 and M364, produced by treating rapeseed with X-ray or ethylmethane sulfonate. M57 had reduced a-linolenic acid while M364 had increased a-linolenic acid. However, the instability of the fatty acid traits between generations was unacceptable for commercial purposes.

Brunklaus-Jung et al. (PI. Breed., 98:9-16, 1987), backcrossed M57 and other rapeseed mutants obtained by mutagenic treatment to commercial varieties. BCₒ and BC₁ of M57 contained 29.4-33.3% of linoleic acid (C_{18:2}) and 4.9-10.8% of a-linolenic acid (C_{18:3}). The oleic acid (C_{18:1}) content was not reported, but by extrapolation could not have exceeded 60%.

Four other lower a-linolenic acid canola lines have been described. Stellar, reported by Scarth et al. (Can. J. Plant Sci., 68:509-511, 1988), is a Canadian cultivar with lower a-linolenic acid (also 3%) derived from M57. Its a-linolenic acid trait was generated by seed mutagenesis. S85-1426, a Stellar derivative with improved agronomic characteristics, also has lower (1.4%) a-linolenic acid (Report of 1990 Canola/Rapeseed Strain Test A, Western Canada Canola Rapeseed Recommending Committee). IXLIN, another lower a-linolenic acid (1.8%) line described by Roy et al. (Plant Breed., 98:89-96, 1987), originated from an interspecific selection. EP-A 323 753 (Allelix) discloses rape plants, seeds, and oil with reduced a-linolenic acid content linked to limitations in the content of oleic acid, erucic acid, and glucosinolate.

Another nutritional aspect of rapeseed, from which canola was derived, is its high (30-55 µmol/g) level of glucosinolates, a sulfur-based compound. When the foliage or seed is crushed, isothiocyanate esters are produced by the action of myrosinase on glucosinolates. These products inhibit synthesis of thyroxine by the thyroid and have other antimetabolic effects (Paul et al., Theor. Appl. Genet. 72:706-709, 1986). Brassica varieties with reduced glucosinolates content (<30 µmol/g defatted meal) were developed to increase the nutritional value of canola meal (Stefansson et al., Can. J. Plant Sci. 55:343-344, 1975). Meal from an ultra-low glucosinolates line, BC86-18, has 2 µmol/g total glucosinolates and significantly improved nutritional quality compared to generic canola meal (Classen, Oral presentation, GCIRC Eighth International Rapeseed Congress, Saskatoon, Saskatchewan, July 9-11, 1991). Neither its fatty acid composition nor its seed glucosinolates profile is known.

There remains a need for an improved canola seed and oil with very low a-linolenic levels in the oil and low glucosinolates in the seed to significantly reduce the need for hydrogenation. The a-linolenic content of such a desirable oil would impart increased oxidative stability, thereby reducing the requirement for hydrogenation and the production of trans fatty acids. The reduction of seed glucosinolates would significantly reduce residual sulfur content in the oil. Sulfur poisons the nickel catalyst commonly used for hydrogenation (Koseoglu et al., Chapter 8, pp. 123-148, In: F. Shahidi, ed. Canola and Rapeseed: Production, Chemistry, Nutrition, and Processing Technology, Van Nostrand Reinhold, New York, 1990). Additionally, oil from a canola variety with low seed glucosinolates would be less expensive to hydrogenate.

### 2. Summary of the Invention

In one aspect, our invention provides a canola oil from Brassica seeds having, following crushing and extraction of said seeds:
an a-linolenic acid content of 7% or less relative to the total fatty acid content of said seeds; and
a sulfur content of less than or equal to 3.0 ppm. The oil can provide a significantly reduced overall room-odor intensity relative to the overall room-odor intensity of generic canola oil, a significant difference in overall room-odor intensity being indicated by a difference of greater than 1.0 obtained in a standardized sensory evaluation [Mounts, J. Am. Oil Chem. Soc., 56:659-663, 1979].

Such oil is obtainable from a Brassica napus canola yielding seed having a total glucosinolates content of about 18 µmol/g or less of defatted, air-dried meal. Crushed seeds of such canola form another aspect of the invention. The seed has an a-linolenic acid content of less than or equal to 7%, more preferably less than or equal to about 4.1% a-linolenic acid (C_{18:3}) relative to total fatty acid content of said seed and a total glucosinolates content of less than 18 wmol/g, more preferably less than or equal to about 15 µmol/g and most preferably less than or equal to 13 gmol/g and belongs to a line in which these traits have been stable for both the generation to which the seed belongs and that of its parent.

Preferably, the oil has an a-linolenic acid content of 1.7% or more relative to the total fatty acid content of said seeds. Especially preferably, the oil has an erucic acid content of 0.5% or less relative to the total fatty acid content of said seeds. Preferably the oil has a linoleic acid content of 25.8% or less relative to the total fatty acid content of said seeds.

The invention also includes a canola oil as defined above having, when hydrogenated, a significantly reduced overall room-odor intensity relative to the overall room-odor intensity of generic canola oil. The canola oil of the invention when non-hydrogenated can be significantly reduced in fishy or acrid odor relative to the fishy or acrid odor of generic canola oil, respectively, such odor being characteristic of Brassica seed oil.

The invention further relates to crushed Brassica seeds comprising an oil fraction and a meal fraction, said oil fraction being as defined above and said meal fraction having a total glucosinolate content of about 18 µmol/g or less of defatted, air-dried meal. Preferably the total glucosinolate content comprises less than about 6.3 µmoi of aliphatic glucosinolates/g defatted, air-dried meal. Preferably the total glucosinolate content comprises less than about 7.2 µmol of indolyl glucosinolates/g of defatted, air-dried meal. The invention further extends to crushed seeds as described above in which said seeds when grown before crushing under varying conditions yielding an oil fraction having an a-linolenic acid content in a range from about 1.7% to about 7%, relative to the total fatty acid content of said seeds; said oil fraction having an overall room-odor intensity [Mounts, J. Am. Oil Chem. Soc., 56:659-663, 1979] that is substantially the same throughout said a-linolenic acid content range, whether the oil is hydrogenated or not.

Viewed from a yet further aspect, the invention provides a method of producing a canola oil as defined above from Brassica seeds, comprising the steps of:
(a) crossing an agronomically elite plant with a plant producing seeds yielding an oil having an a-linolenic acid content of 7% or less relative to the total fatty acid content of said seeds, a sulfur content of less than or equal to 3.0 ppm and a total glucosinolate content of about 18 µmol/g or less of defatted, air-dried meal;
(b) obtaining progeny from said cross;
(c) identifying at least one descendant among said progeny that yields an oil having, following crushing and extraction of seeds of said descendant, an a-linolenic acid content of 7% or less relative to the total fatty acid content of said descendant seeds and a sulfur content of less than or equal to 3.0 ppm, said descendant seeds having a total glucosinolate content of about 18 wmol/g or less of defatted, air-dried meal;
(d) crushing seeds from progeny of said at least one descendant; and
(e) extracting said canola oil from said crushed seeds, said oil having the composition and properties as defined above.

We have located a stable canola line yielding oil having the above-defined properties referred to herein as IMC 01. Seed of this type is deposited with the American Type Culture Collection, 12301 Parklawn Drive, Rockville, MD, USA 20852 and bearing accession number ATCC 40579.

As mentioned hereinbelow, the characteristics of the oil of the invention may vary according to the conditions under which the plants are grown. Thus in a preferred aspect, the seeds for the production of the oil is derived from field-grown Brassica.

### 3. Detailed Description of the Invention

A spring canola (Brassica napus L.) variety was developed with improved sensory characteristics and oxidative stability in the seed oil. This variety, designated IMC 01, has very low levels of a-linolenic acid (CHgCH₂CH=CHCH₂CH=CHCH₂CH=CH(CH₃)_{7COO}H) in the seed oil and very low levels of glucosinolates in the seed. The oil produced from the seed of this variety has very low levels of sulfur and was shown to have significantly improved sensory characteristics over generic canola oils. The IMC 01 is a line in which these traits have been stabilized for both the generation to which the seed belongs and that of its parent generation. Particularly desirable lines of this invention from an agronomic point of view can be derived by conventionally crossing lines producing seeds meeting the definitions of this invention with agronomically well-proven lines such as Westar.

In the context of this disclosure, a number of terms are used. As used herein, a "line" is a group of plants that display little or no genetic variation between individuals for at least one trait. Such lines may be created by several generations of self-pollination and selection, or vegetative propagation from a single parent using tissue or cell culture techniques. As used herein, the terms "cultivar" and "variety" are synonymous and refer to a line which is used for commercial production. "Stability" or "stable" means that with respect to the given component, the component is maintained from generation to generation and, preferably, at least three generations at substantially the same level, e.g., preferably ± 15%, more preferably ± 10%, most preferably ± 5%. The stability may be affected by temperature, location, stress and the time of planting. Comparison of subsequent generations under field conditions should produce the component in a similar manner. "Commercial Utility" is defined as having good plant vigor and high fertility, such that the crop can be produced by farmers using conventional farming equipment, and the oil with the described components can be extracted from the seed using conventional crushing and extraction equipment. To be commercially useful, the yield, as measured by both seed weight, oil content, and total oil produced per acre, is within 15% of the average yield of an otherwise comparable commercial canola variety without the premium value traits grown in the same region. "Agronomically elite" means that a line has desirable agronomic characteristics such as yield, maturity, disease resistance, standability. The amount of fatty acids, such as oleic and linolenic acids, that are characteristic of the oil is expressed as a percentage of the total fatty acid content of the oil. "Saturated fatty acid" refers to the combined content of palmitic acid and stearic acid. "Polyunsaturated fatty acid" refers to the combined content of linoleic and a-linolenic acids. The term "shortening" refers to an oil that is a solid at room temperature. The term "room odor" refers to the characteristic odor of heated oil as determined using the room-odor evaluation method described in Mounts (J. Am. Oil Chem. Soc., 56:659-663, 1979). "Generic canola oil" refers to a composite oil extracted from commercial varieties of rapeseed currently known as of the priority date of this application, which varieties generally exhibited at a minimum 8-10% a-linolenic acid content, a maximum of 2% erucic acid and a maximum of 30 µmol/g total glucosinolate level. The seed from each growing region is graded and blended at the grain elevators to produce an acceptably uniform product. The blended seed is then crushed and refined, the resulting oil being sold for use. Table A shows the distribution of canola varieties seeded as percentage of all canola seeded in Western Canada in 1990.

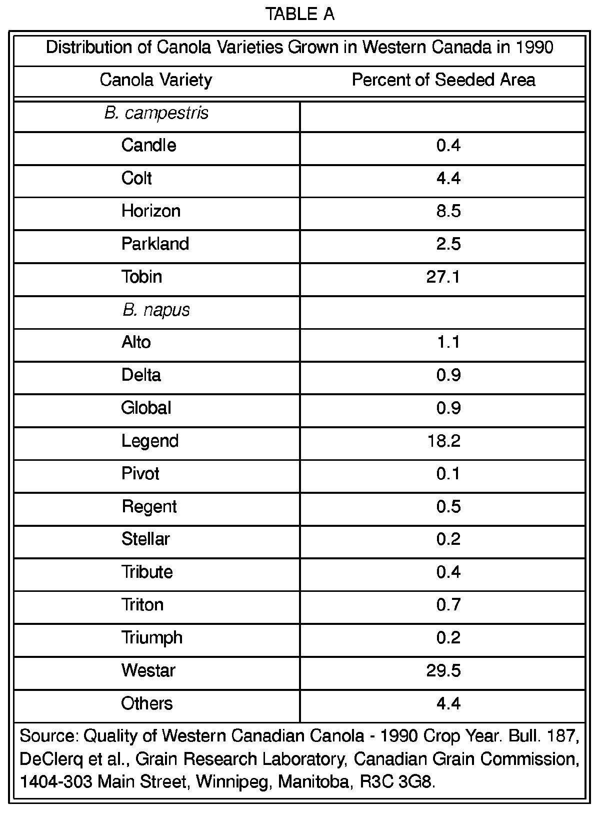

IMC 01 is a very low a-linolenic acid (<4.1% C_{18:3}) line selected during an extensive germplasm screening effort. Its parentage is unknown. IMC 01 was self-pollinated and selected for low a-linolenic acid (<4.1%) over four consecutive generations. At each generation, seeds from individually pollinated plants were analyzed for fatty acid composition. Data showed no genetic segregation for a-linolenic acid content over five generations of self-pollinations (Table I). Breeder seed was derived from a bulk seed increase of selected plants from the fourth self-crossed generation.

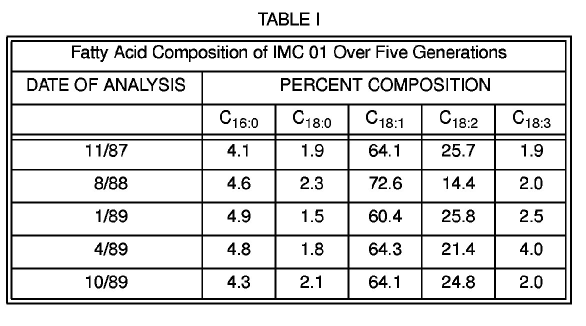

IMC 01 was planted in replicated field trials in North Dakota, South Dakota, Minnesota, Washington, Idaho and Montana in 1989 and 1990, under both irrigated and nonirrigated conditions. These tests showed that the a-linolenic acid content of IMC 01 was sensitive to temperature (Table II). This was further supported by growing IMC 01 under controlled temperature conditions in growth chambers. Whether or not the observed temperature sensitivity of IMC 01 is common to other low a-linolenic acid canola lines is unknown.

A temperature effect on fatty acid compositions has been widely reported in plants, especially oilseed crops (Ren- nie et al., J. Am. Oil Chem. Soc., 66:1622-1624, 1989). These reports describe general temperature effects on fatty acid composition.

Changes in fatty acid content in seed oil under cool temperatures have been documented in plants such as soybean, peanut and sunflower (Neidleman, In: Proceedings of the World Conference on Biotechnology for the Fats and Oils Industry, Applewhite, T H., ed., pp. 180-183. Am. Oil. Chem. Soc. 1987).

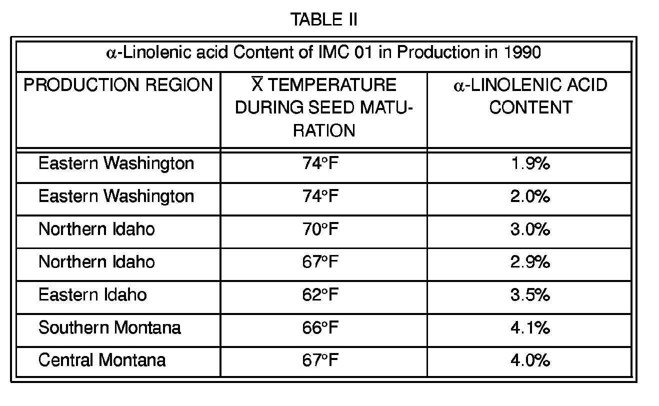

In addition to very low a-linolenic acid, IMC 01 is also characterized by very low levels of glucosinolates. Glucosinolates are sulfur-based compounds common to all Brassica seeds. Glucosinolates exist in aliphatic or indolyl forms. Aliphatic glucosinolates can be analyzed via gas chromatography (GC) (Daun, Glucosinolate analysis of rapeseed (canola), Method of the Canadian Grain Commission, Grain Research Laboratory, Canadian Grain Commission, Winnipeg, 1981). Indolyl glucosinolates have only recently been analyzed via high performance liquid chromatograph (HPLC). Prior to the adoption of the HPLC method, total glucosinolates were calculated by multiplying the aliphatic glucosinolates by a correction factor. Canola quality in the seed is defined as having <30 ₄mol/g of glucosinolates in the defatted meal.

IMC 01 and Westar were tested in five locations in southeastern Idaho in 1990. Three of the locations were irrigated (I) and two were dryland (D) conditions. Table Ilia shows the difference in total aliphatic glucosinolate between IMC 01 and Westar grown at these locations. The aliphatic glucosinolate values are reported as µmol/gm of defatted meal.

The aliphatic glucosinolate content of IMC 01 by location was consistently lower and more stable than that of Westar at all locations tested. The average glucosinolate contents of IMC 01 was 4.9 µMol/gm while Westar was 13.3 µmol/gm. A Least Significant Difference (LSD) test was used to determine if the two were significantly different at all Icoations. IMC 01 was found to be significantly different from Westar at a level of P<0.05.

HPLC analysis of IMC 01 vs Westar, the most widely grown spring canola variety in North America, shows that IMC 01 has much lower levels of aliphatic glucosinolates (Table III). No significant differences exist for indolyl glucosinolates. Glucosinolates content is also subject to environment influence, e.g., sulfur fertility and drought stress. However, IMC 01 consistently had the lowest and the most stable aliphatic glucosinolates levels at all locations tested (Table IV). The locations tested differ in altitude, temperature, fertility irrigation, and other cultural practices. Among the low a-linolenic canola lines for which glucosinates analysis have been performed, IMC 01 has the lowest level of total seed glucosinolates (Table V).

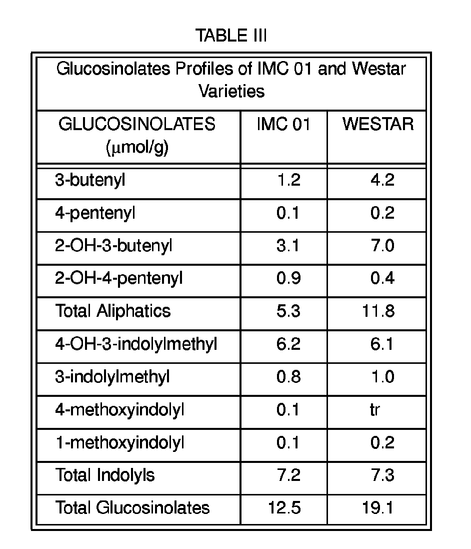

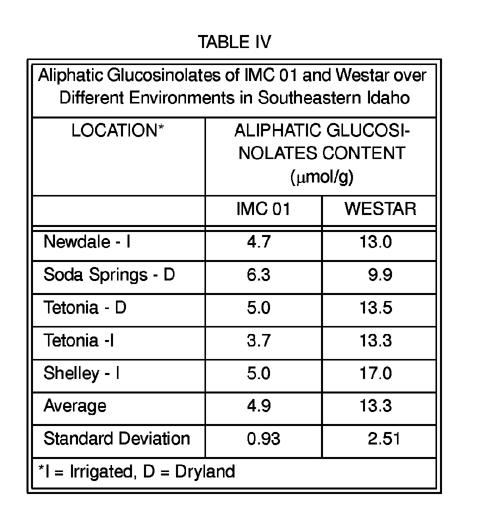

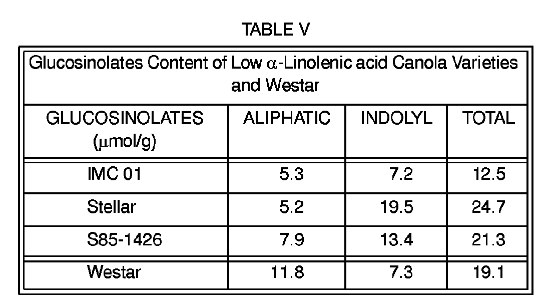

IMC 01 was produced, using normal production practices for spring canola, in Idaho and North Dakota in 1988, in Idaho, Washington State and Montana in 1989, in Idaho, Washington State, Montana, Oregon, and Wyoming in 1990. When grown in suitable environments, where the average daily temperature (high temperature plus low temperature divided by 2) exceeds 20°C, the oil contains <4.1 % a-linolenic acid. As an example, a normal fatty acid profile was produced at Casselton, North Dakota. The crop produced in Ashton, Idaho, was subject to extremely cool conditions and had higher levels of a-linolenic acid. The crops obtained from the field tests were crushed and processed to produce refined, bleached and deodorized (RBD) canola oil at the Protein, Oil, Starch (POS) Pilot Plant in Saskatoon, Saskatchewan. A method of bleaching canola oil is provided in the AOCS' Recommended Practice Cc 8a-52 (AOCS Methods and Standard Practices, 4th Edition (1989)). A method for the refining of crude oils is provided in the AOCS Practice Cc 9a-52 (AOCS Methods and Standard Practices, 4th Edition (1989)). The oils were tested at the Vegetable Oil Research Laboratory, U.S.D.A./Northern Regional Research Center, for organoleptic and sensory characteristics.

Testing to assure that desirable sensory characteristics are obtained in the Brassica napus variety was essential. The evaluation of odors has been conducted in a variety of ways on low a-linolenic acid canola oils. The testing methods are based on the fact that vegetable oils emit characteristic odors upon heating. For example, Prevot et al. (J. Amer. Oil Chemists Soc. 67:161-164, 1990) evaluated the odors of a French rapeseed, "Westar", and "low linolenic" canola oils in a test which attempted to reproduce domestic frying conditions. In these evaluations the test oils were used to fry potatoes and the odors were evaluated by a test panel. The odor tests showed that the "low linolenic" (approximately 3%) line had a significantly higher (more acceptable) odor score than the French rapeseed and "Westar" lines, which were very similar to each other. Eskin et al. (J. Amer. Oil Chemists Soc. 66:1081-1084, 1989) evaluated the odor from canola oil with a low linolenic acid content, a laboratory deodorized sample, and a commercially deodorized sample by sniffing in the presence of the oil itself. These studies demonstrated that a reduction in the linolenic acid content of canola oil from 8-9% to 1.6% reduced the development of heated odor at frying temperatures. However, the odor of the low linolenic acid oil was still unacceptable when heated in air to a majority of the panelists, suggesting that low linolenic acid alone is not sufficient to guarantee acceptable odor.

Mounts (J. Am. Oil Chem. Soc., 56:659-663, 1979) describe a distinct room-odor evaluation method that is used to reproducibly assess the odor characteristics of a cooking oil upon heating. This is the evaluation method of choice owing to its reproducibility and its approximation of odors emitted upon heating the oil. In this method, the oil is heated in a separate chamber and the odor pumped into the room containing the trained evaluators. As noted elsewhere, where the term "room-odor" is used herein, it refers to this method of Mounts. This method is distinct from earlier described tests where the oil and evaluator are within the same room. Such same room testing is referred to as "uncontrolled bench top odor tests" and is considered less accurate and less reliable than the Mounts' room odor evaluation method.

The room-odor characteristics of cooking oils can be reproducibly characterized by trained test panels in room-odor tests (Mounts, J. Am. Oil Chem. Soc. 56:659-663, 1979). A standardized technique for the sensory evaluation of edible vegetable oils is presented in AOCS' Recommended Practice Cg 2-83 for the Flavor Evaluation of Vegetable Oils (Methods and Standard Practices of the AOCS, 4th Edition (1989)). The technique encompasses standard sample preparation and presentation, as well as reference standards and method for scoring oils. When heated, generic canola oil has reduced stability and produces offensive room odors. Refined-Bleached-Deodorized (RBD) canola oil is characterized by a fishy flavor in such tests. This characteristic is commonly ascribed to its high polyunsaturated fatty acid content, particularly a-linolenic acid, relative to other vegetable oils. The individual fragrance notes (odor attributes) of the oils are evaluated by Least Significant Difference Analysis. Notes which differ by greater than 1.0 can be reproducibly measured by a sensory panel. In these tests, IMC 01 oil expressed significantly reduced levels of the offensive odors (Table VI).

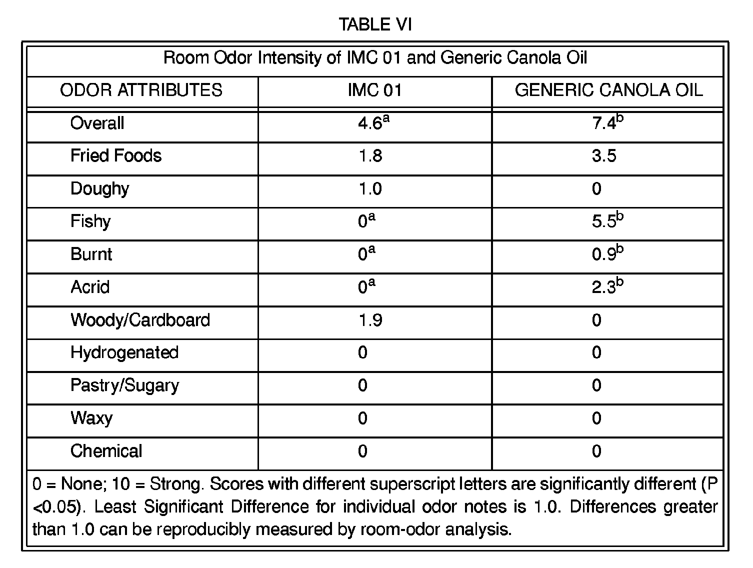

Due to its relatively low stability, canola oil is often hydrogenated for frying. However, hydrogenation produces a characteristic (hydrogenated) room odor which is unacceptable to food manufacturers. Surprisingly, hydrogenated IMC 01 oil also has reduced levels of the characteristic hydrogenated room odor (Table VII). Table VII shows that the overall room-odor intensity of hydrogenated IMC 01 is significantly less than that of hydrogenated generic oil as indicated by a difference is scores of greater than 1.0 in standardized flavor evaluation trials.

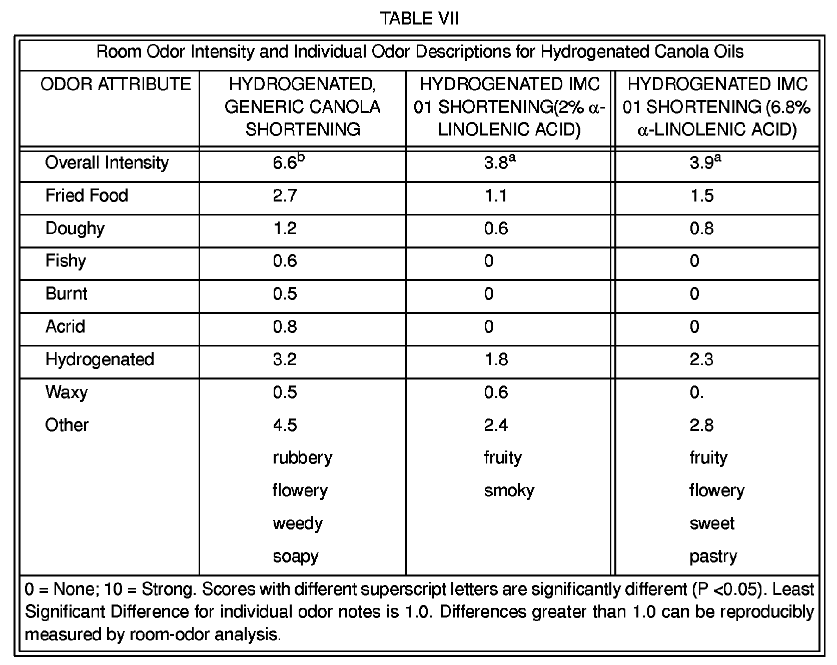

IMC 01 produces an oil which has improved sensory characteristics. Such improvements have been predicted for low a-linolenic acid canola oils (Ulrich et al., J. Am. Oil Chem. Soc., 8:1313-1317, 1988). However, the improved sensory characteristics of IMC 01 appears not to be related solely to its low a-linolenic acid content. Surprisingly, IMC 01 canola oils with both high and low levels of a-linolenic acid showed similar degrees of improvement. Sensory tests have shown that IMC 01 oil maintains its improved quality at both 2% and 6.8% a-linolenic acid.

The very low glucosinolates characteristic of IMC 01 seed is believed to contribute to the improved sensory characteristic of IMC 01 oil. Glucosinolates in the seed are converted to sulfur compounds. Most of the sulfur breakdown products remain in the meal, but some inevitably contaminate the oil. Lower levels of glucosinolates in the seed are believed to result in lower sulfur content in the oil, and this is believed to reduce the objectionable odor characteristics of canola oil (Abraham et al., J. Am. Oil Chem. Soc., 65:392-395, 1988). An analysis of the sulfur content of IMC 01 oil and several generic canola oils has been performed. IMC 01 oil has approximately one-third the sulfur content of leading generic canola oils (Table VIII).

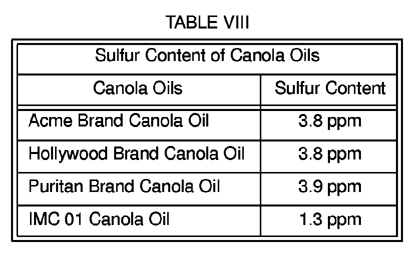

The biochemical, molecular and genetic mechanisms responsible for the room-odor quality of vegetable oils are not fully understood. Improvements in vegetable oil processing technology, i.e., preferential removal of sulfur during processing, less abusive oil extraction procedures, minimal processing, gentler deodorization, etc., may improve the overall quality of vegetable oils, including both sensory and functional characteristics (Daun et al., J. Am. Oil Chem. Soc., 53:169-171, 1976). IMC 01 will benefit from any such processing improvements, and will maintain its improved sensory characteristics over generic canola oil under equivalent processing conditions.

IMC 01 is true breeding as are its progeny. The traits responsible for reduced a-linolenic acid and reduced total glucosinolates in the seed which yield an oil low in sulfur having improved sensory characteristics have a genetic basis. The data presented herein show that these traits are stable under different field conditions. These traits can be removed from the IMC 01 background and are transferred into other backgrounds by traditional crossing and selection techniques.

Crosses have been made with IMC 01 as one parent to demonstrate that the superior IMC 01 quality/sensory traits are transferred along with the superior agronomic traits of another parent such as the Canadian canola line, Westar, into descendants The parent to which IMC 01 is crossed is chosen on the basis of desirable characteristics such as yield, maturity, disease resistance, and standability. Conventional breeding techniques employed in such crossings are well known by those skilled in the art. Thus, a method of using the IMC 01 Brassica na^{p}us is to cross it with agronomically elite lines to produce plants yielding seeds having the characteristics listed above.

The general protocol is:
a. cross IMC 01 to a selected parent;
b. produce a "gametic array" using microsphores of the F₁ plants to produce dihaploid (DH) individuals;
c. field trial DH₂ individuals for yield and select from IMC 01 a-linolenic acid and glucosinolate levels; and
d. test selected individuals for oil quality using RBD oil.

Example 2 is a specific example of such work to develop descendents to IMC 01 which retain the desirable quality traits. The data of Example 2 show that the quality traits of IMC 01 are heritable in such crosses.

The present invention is further defined in the following Examples, in which all parts and percentages are by weight and degrees are Celsius, unless otherwise stated. It should be understood that this Example, while indicating preferred embodiments of the invention, is given by way of illustration only.

### EXAMPLE 1

IMC 01, originally designated DNAP #336, was grown in a greenhouse in Cinnaminson, New Jersey, over several seasons to select for a stable, very low a-linolenic line. Day/night temperatures from August through December in the greenhouse averaged 80°F/65°F with fluctuations of ± 5°F, 75°F/65°F from January through April, and 85°F/65°F from March through July. The plants were grown in 1-gallon pots under natural day length, except from October through May when the plants received 14 hours of supplemental lighting. Flowering racemes were covered with paper bags to prevent cross-pollination, and gently shaken to induce seed set. Watering was decreased as pods reached maturity

For field testing, IMC 01 was planted in multi-location trials and production plots in Montana, Idaho and Washington. The trials were planted in a completely randomized block design with four replications. Each block contained eight plots of 6 meters by 8 rows. IMC 01 was also planted in large acreages (>25 acres) according to standard agronomic procedures for spring canola production, with a minimum 1/2-mile isolation from other Brassica na^{p}us crops. Depending on location, the fields were planted in April or May, and harvested in August or September. Plantings were made on dryland, following both fallow or recrop, or under irrigation. Mature pod samples were taken following swathing for chemical analysis.

For fatty acid analysis, 10-50 seed samples were ground in 15-mL polypropylene tubes and extracted in 1.2 mL 0.25 N KOH in 1:1 ether/methanol. The sample was vortexed for 10 sec and heated for 60 sec and in 60°C water bath. Four mL of saturated NaCI and 2.4 mL of iso-octane were added, and the mixture was vortexed again. After phase separation, 600 µL of the upper organic phase was pipetted into individual vials and stored under nitrogen. One pL sample was injected into a Supelco SP-2330 fused silica capillary column (0.25 mm ID, 30 m length, 0.20 pm df, Bellfonte, PA).

The gas chromatograph was set at 180°C for 5.5 min, then programmed for a 2°C/min increase to 212°C, and held at this temperature for 1.5 min. Chromatography settings were: Column head pressure - 15 psi, Column flow (He) - 0.7 mUmin, Auxiliary and Column flow - 33 mUmin, Hydrogen flow = 33 mL/min, Air flow - 400 mUmin, Injector temperature - 250°C, Detector temperature - 300°C, Split vent - 1/15.

A standard industry procedure for HPLC analysis of glucosinolates was used to analyze the glucosinolates composition of the seed (Daun et al., In: Glucosinolate Analysis of Rapeseed (Canola). Method of the Canadian Grain Commission, Grain Research Laboratory, 1981).

IMC 01 seed was harvested and processed to produce refined, bleached and deodorized (RBD) oil. Some oil was hydrogenated after refining, bleaching and deodorization, then redeodorized.

Before extraction, the seed was tempered to adjust the moisture content to 9% and flaked to 0.38 to 0.64 cm in a ribbon blender. The flakes were cooked in a stack cooker at 82.8°C for 30 min (8.5% moisture) and pre-pressed with vertical and horizontal bar spacings set to 0.031 cm, vertical shaft speed at 40 rpm and horizontal shaft at 25 rpm. The press cake was extracted in a Crown Model 2 extractor at 37.3 kg and hexane extracted with a 2:1 solvent to solids ratio.

The crude oil was alkali refined at 65°C-70°C for 30 min with 0.2% to 85% phosphoric acid, then mixed with sodium hydroxide to neutralize free fatty acids. Soaps were removed with a water wash (65°C water, 5 min) and the oils bleached with .75% each by weight of Clarion and Acticil bleaching earths for 30 min to remove color bodies. The resulting oil contained no peroxides, 0.08% free fatty acids, and had a Gardner color of 10-.

The oil was continuously deodorized at 265°C at 300 kg/h. The steam rate was 1% of feed rate. The deodorized oil was preheated to 68-72°C prior to deaeration. RBD oil was stored in food grade plastic drums or pails at 4°C under nitrogen prior to testing.

For hydrogenation, RBD oil was heated to 350°F under vacuum in a stainless steel pressure reactor. A 0.5% sulfur- poisoned nickel catalyst, Englehardt SP-7, was added to the oil at 80.1°C, and hydrogen gas was introduced at 40 psi. Periodic samples were analyzed until an oil with a 30.5°C melting point was achieved. The hydrogenated oil was redeodorized and stored by the methods described previously.

The RBD and hydrogenated oil samples were analyzed for room-odor characteristics by a trained test panel in comparison with a generic, commercially available RBD canola oil (Proctor & Gamble) and generic, commercially available hydrogenated canola shortening as described previously. The testing protocol used is described in Mounts (J. Am. Oil Chem. Soc. 56:659-663, 1979) which is hereby incorporated by reference. The testing controlled for temperature of the oil, distance from the oil and room volume, and required that the oil was heated in a separate chamber and pumped into the room containing the trained panelist.

Specifically, room odor profiles of IMC 01 and a generic canola oil were obtained as follows:

### A. Room Odor Protocol

A 150 mL sample of the selected oil was heated to 190°C for 30 min before the start of each panel test. The oil was maintained at this temperature throughout each session. For each session a fresh oil sample was used.

Panelists visited each odor room for approximately 15 sec. A five min rest was required between visits. Visitation to each odor room was randomized among the panelists.

The trained panelists judged the room odor for intensity of odor, quality of odor, and odor attributes. The intensity was ranked as: 0-4 weak, 5-7 moderate, and 8-10 strong. The quality of odor was judged as: 0-1 bad, 2-3 poor, 4-6 fair, 7-8 good, and 9-10 excellent. The odor attributes were ranked as: 0-1 bland, 2-4 weak, 5-7 moderate, and 8-10 strong. The flavor attributes were fried, painty, fishy, hydrogenated, burnt, cardboard, metallic, rubbery, waxy, buttery, and nutty

### B. Generic Oil - IMC 01 Profile Comparison

A generic, commercially available canola oil (Proctor & Gamble) was used in the IMC room odor tests as the standard or generic canola oil. In a comparative test, the standard canola oil was significantly (P<0.05) higher in room odor intensity than IMC 01 (Table IX). The standard canola oil odor was of "moderate" intensity while the IMC 01 was considered "weak". The overall quality of the IMC 01 room odor was significantly (P<0.05) better than the standard canola oil. The standard canola oil had significantly (P<0.05) higher intensities for fried, painty, and cardboard odors than the IMC 01 oil.

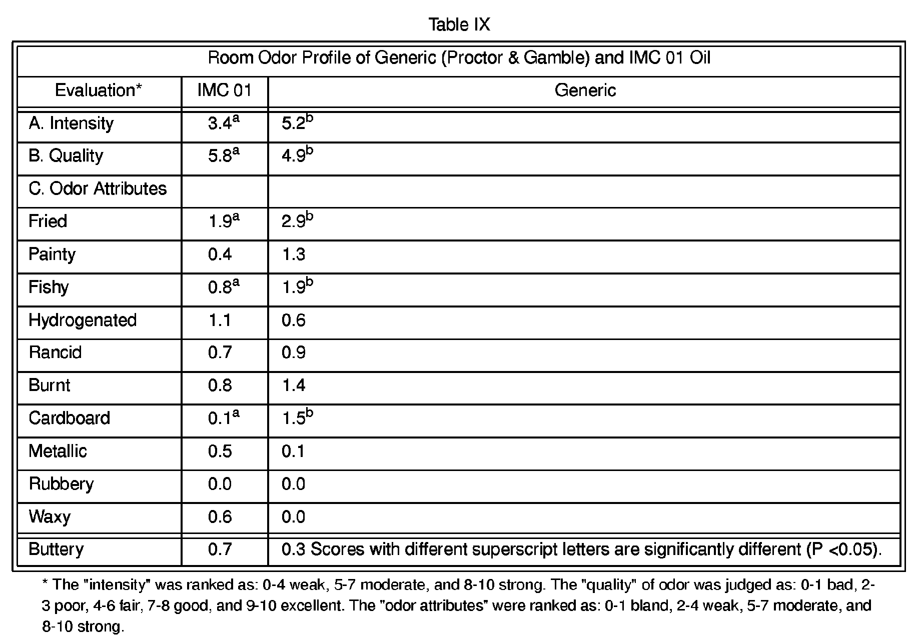

Pilot plant-processed samples of Example 1 generic canola (low erucic acid rapeseed) oil and oil from IMC 01 canola with the fatty acid compositions modified by mutation breeding and/or hydrogenation were evaluated for frying stability. a-linolenic acid contents were 10.1% for generic canola oil, 1.7% for canola modified by breeding (IMC 01) and 0.8% and 0.7% for IMC 01 oils modified by breeding and hydrogenation. The IMC 01 modified oils had significantly (P <0.05) less room odor intensity that the generic canola oil after initial heating tests at 190°C as judged by a sensory panel under conditions of AOCS Cg 2-83. The generic canola oil had significantly higher intensities for fishy, burnt, rubbery, smoky, and acrid odors than the modified oils. Foam heights of the modified oils were significantly (P <0.05) less than those of the generic oil after 20, 30 and 40 hrs of heating and frying at 190°C. The flavor quality of french fried potatoes was significantly (P <0.05) better for all the potatoes fried in modified oils than those fried in generic canola oil. The potatoes fried in generic canola oil were described by the sensory panel as fishy. No off-flavors were detected in potatoes fried in the modified oils.

### EXAMPLE 2

This Example demonstrates that the traits of very low a-linolenic acid and very low glucosinolate content are transferred to IMC 01 progeny.

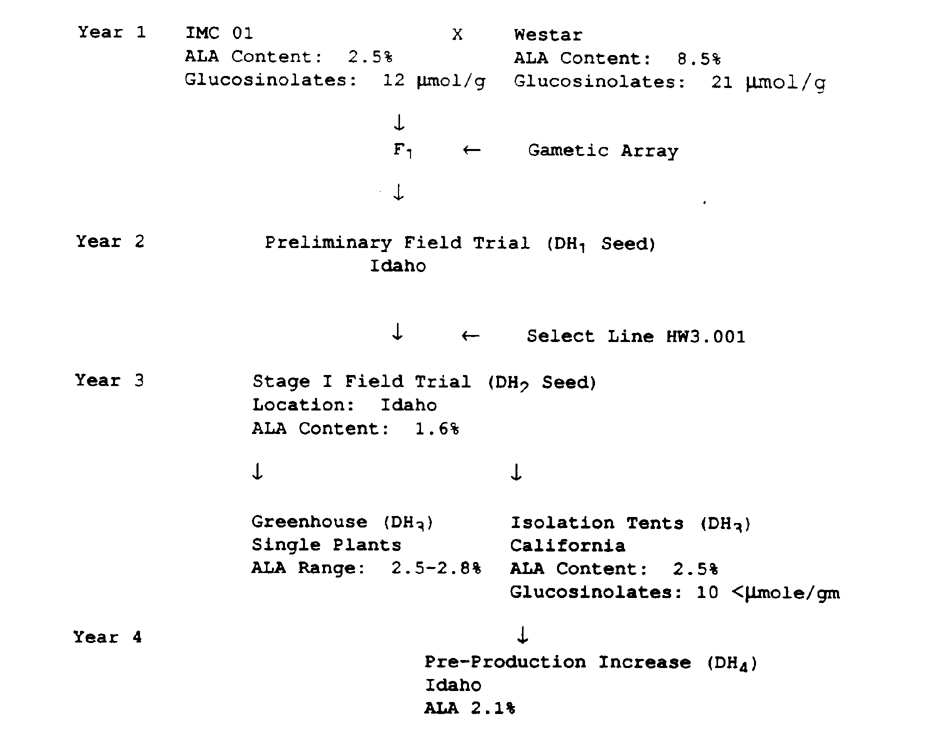

The pre-production will be crushed and the oil refined for quality.

Once a canola line has been stabilized, fully conventional methods of plant biotechnology, breeding and selection are used to further enhance, for example, the agronomic properties of the resultant line in order to improve important factors such as yield, hardiness, etc. Such techniques are also well known and include, e.g., somaclonal variation, seed mutagenesis, anther and microspore culture, protoplast fusion, etc. See, e.g., Brunklaus-Jung et al., PI. Breed., 98:9-16, 1987; Hoffmann et al., Theor. Appl. Genet., 61, 225-232 (1982), each herein incorporated by reference).

A deposit of seed designated IMC 01 has been made in the American Type Culture Collection (ATCC) depository (Rockville, MD 20852) and bears accession number ATCC 40579. The deposit was made on 2 March 1989 under conditions complying with the requirements of the Budapest Treaty

## Claims

1. A canola oil from Brassica seeds having, following crushing and extraction of said seeds:
an a-linolenic acid content of 7% or less relative to the total fatty acid content of said seeds; and
a sulfur content of less than or equal to 3.0 ppm.

2. The oil of claim 1, which has a significantly reduced overall room-odor intensity relative to the overall room-odor intensity of generic canola oil, a significant difference in overall room-odor intensity being indicated by a difference of greater than 1.0 obtained in a standardized sensory evaluation [Mounts, J. Am. Oil Chem. Soc., 56:659-663, 1979].

3. The oil of claim 2 wherein said oil has, when hydrogenated, said significantly reduced overall room-odor intensity relative to the overall room-odor intensity of hydrogenated generic canola oil.

4. The oil of claim 2, said oil having a significantly reduced fishy odor intensity relative to the fishy odor intensity of generic canola oil, a significant difference in fishy odor intensity being indicated by a difference of greater than 1.0 obtained in a standardized sensory evaluation.

5. The oil of claim 2, said oil having a significantly reduced acrid odor intensity relative to the acrid odor intensity of generic canola oil, a significant difference in acrid odor intensity being indicated by a difference of greater than 1.0 obtained in a standardized sensory evaluation.

6. The oil of any of the preceding claims extracted from seeds having, following crushing and extraction of said seeds, an a-linolenic acid content of 4.1% or less relative to the total fatty acid content of said seeds.

7. The oil of any of the preceding claims extracted from seeds having, following crushing and extraction of said seeds, an a-linolenic acid content of 1.7% or more relative to the total fatty acid content of said seeds.

8. The oil of any of the preceding claims extracted from seeds having, following crushing and extraction of said seeds, an erucic acid content of 0.5% or less relative to the total fatty acid content of said seeds.

9. The oil of any of the preceding claims extracted from seeds having, following crushing and extraction of said seeds, a linoleic acid content of 25.8% or less relative to the total fatty acid content of said seeds.

10. The oil of any of the preceding claims wherein said Brassica seeds are from field-grown Brassica.

11. Crushed Brassica seeds comprising an oil fraction and a meal fraction, said oil fraction being as defined in any of claims 1-9 and said meal fraction having a total glucosinolate content of about 18 pmol/g or less of defatted, air-dried meal.

12. Crushed seeds of claim 11, wherein said total glucosinolate content comprises less than about 6.3 µmol of aliphatic glucosinolates/g defatted, air-dried meal.

13. Crushed seeds of claim 11 or 12, wherein said total glucosinolate content comprises less than about 7.2 pmol of indolyl glucosinolates/g of defatted, air-dried meal.

14. Crushed seeds of claim 11, 12 or 13, wherein said seeds are Brassica napus seeds.

15. Crushed seeds of any of claims 11 to 14, said seeds when grown before crushing under varying conditions yielding an oil fraction having an a-linolenic acid content in a range from about 1.7% to about 7%, relative to the total fatty acid content of said seeds; said oil fraction having an overall room-odor intensity [Mounts, J. Am. Oil Chem. Soc., 56:659-663, 1979] that is substantially the same throughout said a-linolenic acid content range.

16. Crushed seeds of claim 15 yielding an oil fraction having, when hydrogenated, an overall room-odor intensity that is substantially the same throughout said a-linolenic acid content range.

17. Crushed seeds of any of claims 11 to 16, wherein the oil fraction has an a-linolenic acid content of 4.1% or less relative to the total fatty acid content of said seeds.

18. Crushed seeds of any of claims 11 to 17, wherein the meal fraction has a total glucosinolate content of less than or equal to 13µmol/g of defatted, air-dried meal.

19. Crushed seeds of any of claims 11 to 18, wherein said seeds are from field-grown Brassica.

20. A method of producing a canola oil as defined in claim 1 from Brassica seeds, comprising the steps of:
(a) crossing an agronomically elite plant with a plant producing seeds yielding an oil having an a-linolenic acid content of 7% or less relative to the total fatty acid content of said seeds, a sulfur content of less than or equal to 3.0 ppm and a total glucosinolate content of about 18 wmol/g or less of defatted, air-dried meal;
(b) obtaining progeny from said cross;
(c) identifying at least one descendant among said progeny that yields an oil having, following crushing and extraction of seeds of said descendant, an a-linolenic acid content of 7% or less relative to the total fatty acid content of said descendant seeds and a sulfur content of less than or equal to 3.0 ppm, said descendant seeds having a total glucosinolate content of about 18 gmollg or less of defatted, air-dried meal;
(d) crushing seeds from progeny of said at least one descendant; and
(e) extracting said canola oil from said crushed seeds, said oil having the composition and properties defined in any of claims 1-9.

21. The method of claim 20, wherein said progeny seeds have the properties defined in any of claims 11 to 19.

22. A method of producing a canola oil as defined in any of claims 1 to 9 which comprises crushing seeds of Brassica as defined in any one of claims 11 to 19 and extracting said oil.

## Patentansprüche

1. Canolaöl aus Brassica-Samen, das nach Zerkleinerung und Extraktion der Samen
einen a-Linolensäuregehalt von 7 % oder weniger, bezogen auf den Gesamtfettsäuregehalt der Samen; und
einen Schwefelgehalt von weniger als oder gleich 3,0 ppm aufweist.

2. Öl nach Anspruch 1, das, bezogen auf die Gesamtraumgeruchsintensität von generischem Canolaöl, eine signifikant verringerte Raumgeruchsintensität aufweist, wobei ein signifikanter Unterschied der Gesamtraumgeruchsintensität durch einen Unterschied von mehr als 1,0, bestimmt durch eine standardisierte sensorische Bewertung [Mounts, J. Am. Oil Chem. Soc., 56:659-663, 1979], angezeigt wird.

3. Öl nach Anspruch 2, das nach Hydrierung im Vergleich zur Gesamtraumgeruchsintensität von generischem, hydriertem Canolaöl die erwähnte, signifikant verringerte Raumgeruchsintensität aufweist.

4. Öl nach Anspruch 2, das, im Vergleich zur Fischgeruchsintensität von generischem Canolaöl, einen signifikant weniger intensiven Fischgeruch aufweist, wobei ein signifikanter Unterschied der Intensität des Fischgeruchs durch einen Unterschied von mehr als 1,0, bestimmt durch eine standardisierte sensorische Bewertung, angezeigt wird.

5. Öl nach Anspruch 2, das, im Vergleich zur Intensität des stechenden Geruchs von generischem Canolaöl, einen signifikant weniger intensiven stechenden Geruch aufweist, wobei ein signifikanter Unterschied der Intensität des stechenden Geruchs durch einen Unterschied von mehr als 1,0, bestimmt durch eine standardisierte sensorische Bewertung, angezeigt wird.

6. Öl nach einem der vorhergehenden Ansprüche, das aus Samen extrahiert wurde und nach Zerkleinerung und Extraktion der Samen einen a-Linolensäuregehalt von 4,1 % oder weniger, bezogen auf den Gesamtfettsäuregehalt der Samen, aufweist.

7. Öl nach einem der vorhergehenden Ansprüche, das aus Samen extrahiert wurde und nach Zerkleinerung und Extraktion der Samen einen a-Linolensäuregehalt von 1,7 % oder mehr, bezogen auf den Gesamtfettsäuregehalt der Samen, aufweist.

8. Öl nach einem der vorhergehenden Ansprüche, das aus Samen extrahiert wurde und nach Zerkleinerung und Extraktion der Samen einen Erucasäuregehalt von 0,5 % oder weniger, bezogen auf den Gesamtfettsäuregehalt der Samen, aufweist.

9. Öl nach einem der vorhergehenden Ansprüche, das aus Samen extrahiert wurde und nach Zerkleinerung und Extraktion der Samen einen Linolsäuregehalt von 25,8 % oder weniger, bezogen auf den Gesamtfettsäuregehalt der Samen, aufweist.

10. ÖI nach einem der vorhergehenden Ansprüche, worin die Brassica-Samen von Brassica aus Feldanbau stammen.

11. Zerkleinerte Brassica-Samen, umfassend eine Ölfraktion und eine Mehlfraktion, wobei die Ölfraktion wie in den Ansprüchen 1 bis 9 definiert ist und die Mehlfraktion einen Glucosinolatgesamtgehalt an entfettetem, luftgetrocknetem Mehl von etwa 18 µmol/g oder weniger aufweist.

12. Zerkleinerte Samen nach Anspruch 11, worin der Glucosinolatgesamtgehalt weniger als etwa 6,3 µmol aliphatische Glucosinolate/g entfettetem, getrocknetem Mehl umfaßt.

13. Zerkleinerte Samen nach Anspruch 11 oder 12, worin der Glucosinolatgesamtgehalt weniger als etwa 7,2 µmol Indolylglucosinolate/g entfettetem, getrocknetem Mehl beträgt.

14. Zerkleinerte Samen nach Anspruch 11, 12 oder 13, wobei die Samen Brassica napus-Samen sind.

15. Zerkleinerte Samen nach einem der Ansprüche 11 bis 14, wobei die Samen, wenn sie vor dem Zerkleinern unter verschiedenen Bedingungen kultiviert wurden, eine Ölfraktion mit einem a-Linolensäuregehalt im Bereich von etwa 1,7 % bis etwa 7 %, bezogen auf den Gesamtfettsäuregehalt der Samen, ergeben; wobei die Gesamtraumgeruchsintensität der Ölfraktion [Mounts, J. Am. Oil Chem. Soc., 56:659-663, 1979] über den gesamten Bereich des a-Linolensäuregehaltes im wesentlichen gleichbleibt.

16. Zerkleinerte Samen nach Anspruch 15, die eine Ölfraktion ergeben, deren Gesamtraumgeruchsintensität nach Hydrierung über den gesamten Bereich des a-Linolensäuregehaltes im wesentlichen gleichbleibt.

17. Zerkleinerte Samen nach einem der Ansprüche 11 bis 16, worin die Ölfraktion einen a-Linolensäuregehalt von 4,1 % oder weniger, bezogen auf den Gesamtfettsäuregehalt der Samen, aufweist.

18. Zerkleinerte Samen nach einem der Ansprüche 11 bis 17, worin die Mehlfraktion einen Glucosinolatgesamtgehalt von weniger als oder gleich 13 gmol/g entfettetem, luftgetrocknetem Mehl aufweist.

19. Zerkleinerte Samen nach einem der Ansprüche 11 bis 18, wobei die Samen von Brassica aus Feldanbau stammen.

20. Verfahren zur Herstellung von Canolaöl nach Anspruch 1 aus Brassica-Samen, wobei man:
(a) eine agronomische Elitepflanze mit einer Pflanze kreuzt, die ein Öl mit einem a-Linolensäuregehalt von 7 % oder weniger, bezogen auf den Gesamtfettsäuregehalt der Samen, einem Schwefelgehalt von weniger als oder gleich 3,0 ppm und einem Gesamtglucosinatgehalt von etwa 18 gmol/g entfettetem, luftgetrocknetem Mehl oder weniger ergibt;
(b) die Nachkommenschaft dieser Kreuzung gewinnt;
(c) wenigstens einen Abkömmling unter dieser Nachkommenschaft identifiziert, der ein Öl ergibt, welches nach dem Zerkleinern und Extrahieren der Samen des Abkömmlings einen a-Linolensäuregehalt von 7 % oder weniger, bezogen auf den Gesamtfettsäuregehalt der Samenabkömmlinge, und einen Schwefelgehalt von weniger als oder gleich 3,0 ppm aufweist, wobei der Gesamtglucosinatgehalt der Samenabkömmlinge etwa 18 µmol/g entfettetem, luftgetrocknetem Mehl oder weniger beträgt;
(d) die Samen der Nachkommenschaft wenigstens eines Abkömmlings zerkleinert; und
(e) das Canolaöl aus den zerkleinerten Samen gewinnt, wobei Zusammensetzung und Eigenschaften des Öls wie in den Ansprüchen 1 bis 9 definiert ist.

21. Verfahren nach Anspruch 20, wobei die Samenabkömmlinge die in den Ansprüchen 11 bis 19 definierten Eigenschaften besitzen.

22. Verfahren zur Herstellung eines Canolaöls nach einem der Ansprüche 1 bis 9, umfassend die Zerkleinern von Brassica-Samen nach einem der Ansprüche 11 bis 19 und die Extraktion des Öls.

## Revendications

1. Huile de canola de graines de Brassica ayant, après broyage et extraction desdites graines :
une teneur en acide a-linolénique de 7 % ou moins par comparaison à la teneur totale en acides gras desdites graines ; et
une teneur en soufre inférieure ou égale à 3,0 ppm.

2. Huile selon la revendication 1, laquelle a une intensité d'odeur ambiante globale significativement réduite par rapport à l'intensité d'odeur ambiante globale de l'huile de canola générique, une différence significative d'intensité d'odeur ambiante globale étant indiquée par une différence supérieure à 1,0 obtenue dans une évaluation sensorielle standardisée [Mounts, J. Am. Oil Chem. Soc., 56:659-663, 1979].

3. Huile selon la revendication 2, où ladite huile a, lorsqu'elle est hydrogénée, ladite intensité d'odeur ambiante globale significativement réduite par rapport à l'intensité d'odeur ambiante globale de l'huile de canola générique hydrogénée.

4. Huile selon la revendication 2, ladite huile ayant une intensité d'odeur de poisson significativement réduite par rapport à l'intensité d'odeur de poisson de l'huile de canola générique, une différence significative d'intensité d'odeur de poisson étant indiquée par une différence supérieure à 1,0 obtenue dans une évaluation sensorielle standardisée.

5. Huile selon la revendication 2, ladite huile ayant une intensité d'odeur âcre significativement réduite par rapport à l'intensité d'odeur âcre de l'huile de canola générique, une différence significative d'intensité d'odeur âcre étant indiquée par une différence supérieure à 1,0 obtenue dans une évaluation sensorielle standardisée.

6. Huile selon l'une quelconque des revendications précédentes, extraite de graines ayant, après broyage et extraction desdites graines, une teneur en acide a-linolénique de 4,1 % ou moins par rapport à la teneur totale en acides gras desdites graines.

7. Huile selon l'une quelconque des revendications précédentes, extraite de graines ayant, après broyage et extraction desdites graines, une teneur en acide a-linolénique de 1,7 % ou plus par rapport à la teneur totale en acides gras desdites graines.

8. Huile selon l'une quelconque des revendications précédentes, extraite de graines ayant, après broyage et extraction desdites graines, une teneur en acide érucique de 0,5 % ou moins par rapport à la teneur totale en acides gras desdites graines.

9. Huile selon l'une quelconque des revendications précédentes, extraite de graines ayant, après broyage et extraction desdites graines, une teneur en acide linoléique de 25,8 % ou moins par rapport à la teneur totale en acides gras desdites graines.

10. Huile selon l'une quelconque des revendications précédentes, où lesdites graines de Brassica reviennent de Brassica cultivé en plein champ.

11. Graines de Brassica broyées, comprenant une fraction d'huile et une fraction de farine, ladite fraction d'huile étant telle que définie selon l'une quelconque des revendications 1 à 9 et ladite fraction de farine ayant une teneur totale en glucosinolates d'environ 18 µmol ou moins par gramme de farine dégraissée, séchée à l'air.

12. Graines broyées selon la revendication 11, où ladite teneur totale en glucosinolates comprend moins d'environ 6,3 I-Lmol de glucosinolates aliphatiques par gramme de farine dégraissée, séchée à l'air.

13. Graines broyées selon la revendication 11 ou 12 où ladite teneur totale en glucosinolates comprend moins d'environ 7,2 I-Lmol de glucosinolates indolyliques par gramme de farine dégraissée, séchée à l'air.

14. Graines broyées selon la revendication 11, 12 ou 13, où lesdites graines sont des graines de Brassica na^{p}us.

15. Graines broyées selon l'une quelconque des revendications 11 à 14, où lesdites graines, cultivées avant broyage dans des conditions variables, donnent une fraction d'huile ayant une teneur en acide a-linolénique comprise dans la gamme d'environ 1,7 % à environ 7 %, par rapport à la teneur totale en acides gras desdites graines ; ladite fraction d'huile ayant une intensité d'odeur ambiante globale [Mounts, J. Am. Oil chem. Soc., 56:659-663, 1979] qui est sensiblement identique pour toute ladite gamme de teneurs en acide a-linolénique.

16. Graines broyées selon la revendication 15, donnant une fraction d'huile ayant, après hydrogénation, une intensité d'odeur ambiante globale sensiblement identique pour toute ladite gamme de teneurs en acide a-linolénique.

17. Graines broyées selon l'une quelconque des revendications 11 à 16, où la fraction d'huile a une teneur en acide a-linolénique de 4,1 % ou moins par rapport à la teneur totale en acides gras desdites graines.

18. Graines broyées selon l'une quelconque des revendications 11 à 17, où la fraction de farine a une teneur totale en glucosinolates inférieure ou égale à 13 gmol/g de farine dégraissée, séchée à l'air.

19. Graines broyées selon l'une quelconque des revendications 11 à 18, où lesdites graines proviennent de Brassica cultivé en plein champ.

20. Procédé de production d'une huile de canola selon la revendication 1 à partir de graines de Brassica, comprenant les étapes consistant à :
(a) croiser une plante agronomiquement élite avec une plante produisant des graines donnant une huile ayant une teneur en acide a-linolénique inférieure ou égale à 7 % par rapport à la teneur totale en acides gras desdites graines, une teneur en soufre inférieure ou égale à 3,0 ppm et une teneur totale en glucosinolates inférieure ou égale à environ 18 µmol/g de farine dégraissée, séchée à l'air ;
(b) obtenir la descendance dudit croisement ;
(c) identifier parmi ladite descendance au moins un descendant donnant une huile ayant, après broyage et extraction des graines dudit descendant, une teneur en acide a-linolénique inférieure ou égale à 7 % par rapport à la teneur totale en acides gras desdites graines du descendant et ayant une teneur en soufre inférieure ou égale à 3,0 ppm, lesdites graines du descendant ayant une teneur totale en glucosinolates inférieure ou égale à environ 18 µmol/g de farine dégraissée, séchée à l'air ;
(d) broyer les graines de la descendance dudit au moins un descendant ; et
(e) extraire ladite huile de canola desdites graines broyées, ladite huile ayant la composition et les propriétés selon l'une quelconque des revendications 1 à 9.

21. Procédé selon la revendication 20, où les graines de ladite descendance ont les propriétés définies selon l'une quelconque des revendications 11 à 19.

22. Procédé de production d'une huile de canola selon l'une quelconque des revendications 1 à 9, lequel comprend le broyage de graines de Brassica selon l'une quelconque des revendications 11 à 19 et l'extraction de ladite huile.
